# EUROPÄISCHE PATENTSCHRIFT

(11) **EP 0 492 494 B1**
(45) Veröffentlichungstag und Bekanntmachung des Hinweises auf die Patenterteilung: **31.07.1996**
(21) Anmeldenummer: 91121929.3
(22) Anmeldetag: 20.12.1991
(51) Int. Cl.: C07C 227/18

(54) **Verfahren zur Herstellung von Procain**
Process for the preparation of procaine
Procédé pour la préparation de procaine

(30) Priorität: 21.12.1990 DE 4041179
(43) Veröffentlichungstag der Anmeldung: 01.07.1992
(73) Patentinhaber: HOECHST AKTIENGESELLSCHAFT, 65926 Frankfurt am Main (DE)
(72) Erfinder: Jürgens, Ernst A., W-6232 Bad Soden am Taunus (DE)

(56) Entgegenhaltungen:
- AT-B- 213 874
- US-A- 1 396 913
- US-A- 1 676 470
- US-A- 3 660 411

## Beschreibung

p-Aminobenzoyldiäthylaminoäthylester (Procain) wird gewöhnlich durch Umsetzung von p-Nitrobenzoylchlorid mit Diäthylaminoäthanol und anschließende katalytische Hydrierung hergestellt. Auch die Veresterung von p-Aminobenzoesäure mit Diäthylaminoäthanol unter Verwendung von Schwefelsäure als Katalysator (USP 812554) ist bekannt. In der AT-C-213874 wird die Herstellung aus p-Aminobenzoesäureester durch Umsetzung mit Diäthylaminoäthanol in Gegenwart von Natriumalkoholat (Natrium gelöst in Diäthylaminoäthanol) beschrieben. Auf die laufende Entfernung des bei dieser Reaktion freiwerdenden Alkohols aus dem Reaktionsgemisch wird hingewiesen. Zur Vermeidung von Nebenprodukten sollen 100 °C nicht überschritten werden. Auch beim Abdestillieren des überschüssigen Alkamins soll diese Temperaturgrenze eingehalten werden. Als Ausgangsester werden stets nur die sehr reaktionsfähigen Methylester genannt.

Ausgangsprodukt für Methyl- und Äthylester sind p-Aminobenzoesäure oder p-Nitrobenzoesäure. Zur Erzielung einer guten Ausbeute ist es erforderlich, daß in der Veresterung zur Entfernung des Wassers ein Schleppmittel wie Benzol zugesetzt wird. Überraschend wurde nun festgestellt, daß die o.g. Temperaturgrenzen bei dem weniger reaktionsfähigen Butylester nicht eingehalten werden müssen und die kontinuierliche Entfernung des bei der Umsetzung freiwerdenden Alkohols möglich ist. Anschließend kann auch das überschüssige Alkamin bei wesentlich höheren Temperaturen, untersucht wurden Temperaturen bis zu 165 °C, ohne Ausbeuteeinbuße abdestilliert werden. Hiermit kann unter wesentlich geringerem Vakuum, mit 150 bis 200 mbar gegenüber 35 bis 60 mbar, als in der österreichischen Patentschrift angegeben, gearbeitet werden. Für die großtechnische Ausübung des Verfahrens ist dies von erheblichem Vorteil. Es wurde ferner gefunden, daß der Butylester der p-Amino-benzoesäure bereits in roher Qualität, wie er nach Veresterung der Nitrobenzoesäure und anschließender Reduktion vorliegt, mit Diäthylaminoäthanol in Gegenwart von Natriumalkoholat umgeestert werden kann. Verlustreiche und kostenintensive Zwischenisolierungen entfallen nach diesem erfindungsgemäßen Verfahren. Der Einsatz leicht siedender Alkohole, wie Methanol und Äthanol, verbunden mit kritischen Schleppmitteln, wie Benzol, entfällt. Mit Butanol gelangt ein Lösemittel zum Einsatz, dessen Dampfdruck niedrig liegt, und der demzufolge ein umweltfreundliches Verfahren ohne große Zusatzmaßnahmen ermöglicht.

p-Nitrobenzoesäure wird in Butanol unter Zusatz geringerer Mengen Schwefelsäure verestert und anschließend der Nitroester katalytisch hydriert. Nachdem der Überschuß an Alkohol entfernt ist, erfolgt nach Zusatz von Diäthylaminoäthanol und geringen Mengen Natrium-alkoholat die Umesterung bei 80 bis 150 °C, vorzugsweise bei 80 bis 130, ganzbesonders bevorzugt bei 80 bis 110, insbesondere bei 90-95 °C. Der hierbei freiwerdende Butylalkohol wird im Vakuum kontinuierlich ausgetragen. Nach beendeter Reaktion wird der Katalysator durch Säurezusatz oder Wasser zersetzt. Das überschüssige Diäthylaminoäthanol wird i.V. bis 150 °C abdestilliert. Der Rückstand wird entweder im Hochvakuum destilliert oder in Butylacetat aufgenommen und das Hydrochlorid des Procains mit Chlorwasserstoff gefällt. Man erhält so über 94 % d.Th. an Procain, bezogen auf den Einsatz an p-Nitrobenzoesäure, welches einer Stufenausbeute von mehr als 98 % d.Th. entspricht.

Der große Vorteil dieses Verfahrens besteht darin, daß bei der Veresterung kein Hilfslösungsmittel zur Wasserentfernung eingesetzt werden muß, Butanol unter Emissionsgesichtspunkten wegen seines wesentlich geringeren Dampfdruckes einfacher zu handhaben ist und die Wiedergewinnungsrate erheblich besser ist. Ferner kann das Verfahren ohne Isolierung von Zwischenstufen durchgeführt werden.

### Beispiel 1

16,7 g p-Nitrobenzoesäure werden in 40 ml Butanol mit 0,02 ml Schwefelsäure zum Sieden erhitzt und das gebildete Reaktionswasser im Abscheider getrennt. Sobald kein Wasser mehr abgeschieden wird und die Umsetzung vollständig ist, wird auf 60 °C abgekühlt und 0,1 g Soda zugesetzt. Nach Zugabe von 0,5 g Pd-Kohle Katalysator wird unter 10 bar Wasserstoffdruck bei 50 bis 100 °C hydriert. Nach beendeter Wasserstoffaufnahme wird auf 50 °C abgekühlt, der pH-Wert überprüft und gegebenenfalls mit wenigen Tropfen Natronlauge auf pH 9 gestellt. Danach destilliert man die Hauptmenge an Butanol i.V. bis 110 °C ab. Nach Zugabe von 40 ml Diäthylaminoäthanol werden zur Erzielung der Wasserfreiheit noch ca. 3 ml abdestilliert, bevor man 0,2 g Natriummethylat zusetzt und bei 100 bis 110 °C den freiwerdenden Butylalkohol kontinuierlich über eine Kolonne im Vakuum bei ca. 150 mbar austrägt. Nach beendeter Reaktion werden 0,2 ml Eisessig zugegeben und das überschüssige Diäthylaminoäthanol i.V. bis zu einer Innentemperatur von 150 °C abdestilliert. Der Rückstand wird entweder im Hochvakuum zur Gewinnung der Procainbase destilliert oder nach dem Abkühlen auf 60 °C in 100 ml Butylacetat und 50 ml Wasser gelöst, der pH durch Zugabe von Natronlauge auf 11 eingestellt, die Phasen getrennt und die organische Phase nochmals mit 50 ml Wasser extrahiert. Durch Einleiten von Chlorwasserstoff in die Butylacetatlösung bis pH 6 wird das Hydrochlorid des Procains gefällt. Die Ausbeute beträgt 26 g oder 95 % d.Th. Umgelöst aus Alkohol schmilzt die Verbindung bei 154 °C.

### Beispiel 2

Das in Beispiel 1 beschriebene Verfahren wird wiederholt, jedoch wird die Umesterungstemperatur auf 90 bis 95 °C reduziert. Innerhalb 4 bis 5 Stunden wird ein vollständiger Umsatz erreicht. Die Aufarbeitung wurde ebenfalls wie in Beispiel 1 beschrieben vorgenommen und lieferte praktisch dieselbe Ausbeute.

## Patentansprüche

1. Verfahren zur Herstellung von Procain, dadurch gekennzeichnet, daß man p-Nitrobenzoesäure in Butanol in an sich bekannter Weise verestert, den Nitroester ohne Zwischenisolierung katalytisch hydriert und die resultierende Hydrierlösung nach Abdestillation der Hauptmenge des verwendeten Alkohols mit Diäthylaminoäthanol in Gegenwart von Alkalialkoholaten bei 80 bis 150 °C und unter einem druck von 150 bis 200 mbar zur Umsetzung bringt, und dabei den freiwerdenden Alkohol im Vakuum laufend abtrennt.

2. Verfahren nach Anspruch 1, dadurch gekennzeichnet, daß man die Umesterung bei Temperaturen von 80 bis 110 °C, vorzugsweise bei 90 bis 95 °C, vornimmt.

## Claims

1. A process for the preparation of procaine, which comprises esterifying p-nitrobenzoic acid in butanol in a manner known per se, catalytically hydrogenating the nitro ester without intermediate isolation, and reacting the resulting hydrogenation solution, after removal by distillation of most of the alcohol used, with diethylaminoethanol in the presence of alkali metal alcoholates at 80 to 150°C and under a pressure of from 150 to 200 mbar, and continuously removing the alcohol liberated thereby in vacuo.

2. The process as claimed in claim 1, wherein the transesterification is carried out at temperatures of 80 to 110°C, preferably at 90 to 95°C.

## Revendications

1. Procédé pour la préparation de la procaïne, caractérisé en ce que l'on estérifie, d'une façon connue en soi, de l'acide p-nitrobenzoïque dans du butanol, on soumet le nitroester, sans isolement intermédiaire, à une hydrogénation catalytique, et, après élimination par distillation de la majeure partie de l'alcool utilisé, on fait réagir la solution d'hydrogénation résultante avec du diéthylaminoéthanol en présence d'alcoolates de métaux alcalins, à 80-150°C et sous une pression de 150 à 200 mbar, et l'alcool libéré est ainsi séparé sous vide en continu.

2. Procédé selon la revendication 1, caractérisé en ce que l'on effectue la transestérification à des températures de 80 à 110°C, de préférence de 90 à 95°C.
